# EUROPÄISCHE PATENTSCHRIFT

(11) **EP 1 622 665 B1**
(45) Veröffentlichungstag und Bekanntmachung des Hinweises auf die Patenterteilung: **18.08.2010**
(21) Anmeldenummer: 04727548.2
(22) Anmeldetag: 15.04.2004
(51) Int. Cl.: A61M 16/04, A61M 16/20

(54) **Vorrichtung zur Beatmung mit einem doppellumigen Endotrachealtubus**
Respiratory device comprising a double lumen endotracheal tube
Dispositif de respiration artificielle comportant un tube endotracheal a deux canaux

(30) Priorität: 30.04.2003 DE 10319384
(43) Veröffentlichungstag der Anmeldung: 08.02.2006
(73) Patentinhaber: Reissmann, Hajo, 22765 Hamburg (DE)
(72) Erfinder: Reissmann, Hajo, 22765 Hamburg (DE)
(74) Vertreter: Hauck Patent- und Rechtsanwälte
(86) Internationale Anmeldenummer: PCT/EP2004/003935
(87) Internationale Veröffentlichungsnummer: WO 2004/096312

(56) Entgegenhaltungen:
- WO-A-02/069786
- WO-A-02/089885
- US-A- 5 606 968
- US-A- 5 957 134

## Beschreibung

Die Erfindung bezieht sich auf eine Vorrichtung zur Beatmung mit einem doppellumigen Endotrachcaltubus (ETT) nach Patentanspruch 1.

Zur Beatmung im Rahmen einer Intensivtherapie oder während einer Narkose werden die Patienten in der überwiegenden Mehrzahl endotracheal intubiert. Dabei wird ein Endotrachealtubus, der in der Regel aus einem Kunststoff oder einem Gummirohr besteht, durch Mund, Nase oder Luftröhrenschnitt ("Tracheotomie") in die Luftröhre eingeführt. Der Endotrachealtubus ist meist am trachealen Ende mit einer aufblasbaren Tubusmanschette ("Cuff") zur Abdichtung der Luftröhre versehen, die die Beatmung mit Überdruck ermöglicht und die Atemwege vor dem Eindringen von Fremdmaterial schützt. Das patientenferne Ende des Endotrachealtubus ist über ein Schlauchsystem an ein Beatmungsgerät ("Respirator") angekoppelt oder ausgangsseitig über ein Rückschlagventil mit der Umgebung verbunden.

Übliche einlumige ETT verfügen über ein Ansatzstück, das in genormter Ausführung ein etwa 10 mm langer Konus mit geringem Konuswinkel (d.h. annähernd zylindrisch) und einem Außendurchmesser von ca. 15 mm ist. Der Inspirationsschlauch und der Exspirationsschlauch des Schlauchsystems werden in einem Y-Verbinder zusammengeführt, in den eine entsprechend konusförmige Aufnahme eingearbeitet ist, so daß der Verbinder abdichtend auf das Ansatzstück aufsetzbar ist.

Bei doppellumigen ETT, wie in der WO 02/089885 A beschrieben, ist herkömmlicherweise jedes Lumen mit einem genormten Ansatzstück verbunden. Die Ansatzstücke werden jeweils über einen geraden Verbinder mit einem Gegen-Konus mit dem Inspirationsschlauch und dem Exspirationsschlauch verbunden. Diese Art der Konnektion ist allerdings unpraktisch und birgt Sicherheitsnachteile:

So verdoppeln zwei Konnektionen die Gefahr der Dekonnektion. Ferner können jeweils zwei identisch dimensionierte Ansatzstücke und Verbinder zu Verwechslungen der beiden Lumina führen. Farbcodierungen oder sonstige Markierungen bieten dagegen nur eingeschränkten Schutz. Eine Verbindung mit einem Beatmungsgerät mit einem konventionellen Y-Verbinder ist nicht ohne weiteres möglich.

Herkömmliche Respiratoren in Verbindung mit einlumigem ETT öffnen im Falle einer Fehlfunktion, die eine reguläre Gaslieferung an den Patienten nicht mehr zuläßt, am Anfang ihres Einatemschenkels ein Sicherheitsventil, über das der Patient, sofern er dazu körperlich in der Lage ist, zumindest Raumluft einatmen kann. Das Ausatemventil ist in der Regel so ausgelegt, daß es in diesem Fehlerfall weiterhin die Ausatmung zuläßt; die beiden Ventile sorgen somit für den nötigen gerichteten Gasstrom bei der Spontanatmung. Bei Einsatz eines Doppel-ETT stünde auch in diesem Fehlerfall für die In- und Exspiration jeweils nur ein Lumen zur Verfügung. Der Patient wäre dann mit relativ hohen Strömungswiderständen konfrontiert, insbesondere wenn das Doppel-ETT eine bei regelrechter Beatmung sinnvolle asymmetrische Ausgestaltung mit engerem inspiratorischem Lumen aufwiese.

Aus WO-A-02/069786 ist eine Vorrichtung zur Beatmung bekannt geworden mit einem doppellumigen Endtrachealtubus mit einem Ansatzstück am patientenfernen Ende, in das die beiden Lumina des Endotrachealtubus hineingeführt sind, wobei sie im Ansatzstück durch eine axiale Trennwand voneinander getrennt sind. Es ist ein Verbinder mit zwei Schlauchanschlüssen vorgesehen, die in einer Aufnahme für das Schlußstück zusammengeführt und durch eine weitere axiale Trennwand voneinander getrennt sind, der mit dem Ansatzstück unter Abdichten der Anlage der Trennwand und der weiteren Trennwand aneinander verbindbar ist. Es ist ferner ein Beatmungsgerät (implizit) vorgesehen und mindestens einem einenends mit einem Schlauchanschluß des Verbinders und anderenends mit dem Beatmungsgerät verbundener Schlauch.

Der Erfindung liegt die Aufgabe zugrunde, im Falle einer Fehlfunktion des Respirators dafür zu sorgen, daß die spontane Atmung des Patienten nicht zu hohen Widerständen ausgesetzt ist.

Die Aufgabe wird durch eine Vorrichtung zur Beatmung mit den Merkmalen des Anspruches 1 gelöst. Vorteilhafte Ausgestaltungen der Vorrichtung sind in den Unteransprüche angegeben.

Die erfindungsgemäße Vorrichtung zur Beatmung hat
- einen doppellumigen Endotrachealtubus mit einem Ansatzstück am patientenfernen Ende, in das die beiden Lumina des Endotrachealtubus hineingeführt sind, wobei sie im Ansatzstück durch eine axiale Trennwand voneinander getrennt sind,
- einen Verbinder mit zwei Schlauchanschlüssen, die in einer Aufnahme für das Anschlußstück zusammengeführt und durch eine weitere axiale Trennwand voneinander getrennt sind, der mit dem Ansatzstück unter abdichtender Anlage der Trennwand und der weiteren Trennwand aneinander verbindbar ist,
- ein Beatmungsgerät und
- mindestens ein einenends mit einem Schlauchanschluß des Verbinders und anderenends mit dem Beatmungsgerät verbundenen Schlauch, wobei
- der Verbinder und/oder das Ansatzstück ein Absperrorgan aufweist/aufweisen, das die beiden Seiten der weiteren Trennwand und/oder der Trennwand überbrückt.

Bei der erfindungsgemäßen Vorrichtung sind die beiden Lumina des Endotrachealtubus durch ein Ansatzstück und einen darauf passenden Verbinder hindurch getrennt mit Schläuchen des Schlauchsystems bzw. nur eingangsseitig mit einem solchen Schlauch und ausgangsseitig über ein Ventil mit der Umgebung verbindbar. Hierzu sind die Lumina in das Ansatzstück hineingeführt und darin durch eine axiale Trennwand voneinander getrennt und die Schlauchanschlüsse in die Aufnahme des Verbinders hineingeführt und darin durch eine weitere axiale Trennwand getrennt. Ferner kommt hierzu bei der Verbindung von Ansatzstück und Verbinder die Trennwand zur abdichtenden Anlage an der weiteren Trennwand, so daß die Trennung der In- und Exspirationsseiten durch die Verbindung von Schlauchsystem und ETT hindurch sichergestellt ist. Da nur eine Verbindung zwischen ETT und Beatmungsgerät vorliegt, ist die Handhabung vereinfacht und das Risiko einer unbeabsichtigten Dekonnektion reduziert. Grundsätzlich ist der Doppel-ETT auch mit einem Respirator mit einem konventionellen Y-Verbinder verbindbar, beispielsweise falls kein Respirator mit einem erfindungsgemäß ausgestatteten Verbinder zur Verfügung steht.

Im Falle einer Fehlfunktion des Respirators kann das Absperrorgan geöffnet werden, um eine Atmung über beide Lumina des Doppel-ETT zu ermöglichen. Damit ist sichergestellt, daß die spontane Atmung des Patienten nicht zu hohen Widerständen ausgesetzt ist, insbesondere wenn der Doppel-ETT asymmetrisch ausgestaltet ist.

Auch aus den folgenden Gründen ist die Freigabe eines Kurzschlusses zwischen den beiden Schlauchschenkeln durch Öffnen des Absperrorgans nützlich: bei einer Leckage irgendwo auf der Strecke zwischen Respirator und Patient versucht der Respirator in druckgeregelten Beatmungs-Modi durch Steigerung der Gaslieferung bis zum technisch möglichen Maximum den Solldruck wiederherzustellen. Bei großen Leckagen, etwa durch eine Diskonnektion, gelingt ihm dies in der Regel nicht. Wenn bei einer Beatmung über Doppel-ETT eine Diskonnektion auf der Exspirationsseite auftritt, erfolgt die kompensatorische Gaslieferung über den Tubus, d. h. über die Trachea des Patienten. Abhängig von Widerstand des exspiratorischen Tubuslumens und der Bauteile zwischen Tubus und Diskonnektion können hierbei unerwünscht hohe Drücke in der Trachea auftreten. In diesem Fehlerfall ist es nützlich, den Kurzschluß zwischen den beiden Schlauchschenkeln herzustellen.

Gemäß einer Ausgestaltung hat das Ansatzstück einen kreisförmigen Außenumfang und die Aufnahme einen entsprechenden Innenumfang, wie bei den genormten Y-Verbindern. Gemäß einer weiteren Ausgestaltung sind das Ansatzstück und die Aufnahme konisch oder zylindrisch.

Gemäß einer Ausgestaltung ist der Verbinder Y-förmig, entsprechend den herkömmliehen Verbindern, oder T-förmig. Grundsätzlich ist es auch möglich, den Verbinder aus einem herkömmlichen Y-Verbinder und einem in dessen Konus eingesetzten, die Trennwand aufweisenden und einen eigenen Konus für die Verbindung mit dem Ansatzstück aufweisenden Adapter zu bilden.

Die Abdichtung zwischen der Trennwand und der weiteren Trennwand kann auf verschiedene Weise hergestellt werden. Hierzu stoßen gemäß einer Ausgestaltung die Trennwände stumpf gegeneinander oder überlappen einander teilweise. Gemäß einer Ausgestaltung sind zwischen den Trennwänden weichelastische Dichtungen wirksam.

Die Trennwände können verschiedene Querschnitte aufweisen. Gemäß einer Ausgestaltung sind die Trennwände plattenförmig oder rohrförmig. Ein konisches bzw. zylindrisches Ansatzstück und eine konische bzw. zylindrische Aufnahme werden durch plattenförmige Trennwände in Lumina mit kreissegmentförmigen Querschnitten geteilt. Rohrförmige Trennwände sind vorzugsweise konzentrisch in Ansatzstück und Aufnahme angeordnet.

Gemäß einer Ausgestaltung weist das Ansatzstück einen Außendurchmesser von etwa 15 mm und die Aufnahme einen entsprechenden Innendurchmesser auf. Gemäß einer anderen Ausgestaltung weist das Ansatzstück einen Innendurchmesser von etwa 22 mm und die Aufnahme einen Außendurchmesser von etwa 22 mm auf. Bei diesen Ausgestaltungen ist der Doppel-ETT mit konventionellen Y-Verbindern an den Schläuchen einsetzbar und die Aufnahme mit konventionellen einlumigen ETTen oder Beatmungsmasken. Hierbei wird die Tatsache ausgenutzt, daß nebeneinander zwei Normmaße zur Konnektion von Schläuchen, Y-Verbindern etc. mit ETTen, Beatmungsmasken etc. bestehen:
- 15 mm, geräteseitig weiblich, patientenseitig männlich - regelhaft, aber nicht ausschließlich verwendet für ETTen;
- 22 mm, geräteseitig männlich, patientenseitig weiblich - regelhaft, aber nicht ausschließlich verwendet für Beatmungsmasken.

Gemäß einer Ausgestaltung hat eine im Ansatzstück angeordnete rohrförmige Trennwand einen Außendurchmesser von etwa 15 mm und eine in der Aufnahme angeordnete weitere rohrförmige Trennwand einen Innendurchmesser von etwa 15 mm und hat das Ansatzstück einen Innendurchmesser von etwa 22 mm und die Aufnahme einen Außendurchmesser von etwa 22 mm. Damit ist patientenseitig (am Doppel-ETT) sowohl ein 15 mm-männlicher als auch ein 22 mm-weiblicher Teil vorhanden. Geräteseitig (am Verbinder) ist sowohl ein 15 mm-weiblicher als auch ein 22 mm-männlicher Teil vorhanden. Dies entspricht handelsüblichen geräteseitigen Verbindern, die vielfach einen Anschlußstutzen haben, der sich an beide Normen hält (d. h. 15 mm Innendurchmesser und 22 mm Außendurchmesser aufweist), um flexibel mit verschiedenen ETTen, Beatmungsmasken etc. einsetzbar zu sein. Erfindungsgemäß sind beide Normen gleichzeitig nutzbar, um die beiden Lumina des ETT über das Innenlumen der rohrförmigen Trennwände und das ringförmige Volumen um die rohrförmigen Trennwände getrennt mit den beiden Schlauchanschlüssen zu verbinden. Obwohl nicht zwingend, ist es günstig, das Innenlumen mit seinem vollen Querschnitt für die Exspiration zu nutzen, das die ungehinderte Passage eines Absaugkatheters zuläßt. Dann fällt dem ringförmigen Außenlumen die Inspiration zu. Ein zusätzlicher Vorteil einer solchen Lösung ist die Rotationssymmetrie, die ein Zusammenstecken in beliebiger Drehrichtung zuläßt.

Eine Verbindung des Verbinders mit einem einlumigen ETT ist möglich, wenn durch geeignete Ausbildung eines Ventils sichergestellt wird, daß das Ringlumen abgesperrt und ein Kurzschluß zwischen den Schlauchschenkeln geöffnet wird. Ferner ist das Ansatzstück mit einem konventionellen Y-Verbinder verbindbar. Wenn beide Lumina des ETT benutzt werden sollen, ist ein Ventil vorzusehen, um einen Kurzschluß zwischen den beiden Lumina zu schalten. Unbedingt erforderlich ist dies jedoch nicht, da - in der vorzugsweise zu realisierenden Ausführung - das zentrale Lumen des Ansatzstückes sich in das weitere Lumen zweier ungleich weiter Lumina des ETT fortsetzt.

Gemäß einer Ausgestaltung hat das Ansatzstück mindestens eine Nut und der Verbinder mindestens einen komplementären Vorsprung oder umgekehrt, die bei Verbindung von Ansatzstück und Verbinder ineinandergreifen. Diese sind so angeordnet, daß sie ein Verbinden von Ansatzstück und Verbinder in nur einer Drehstellung zulassen, so daß eine korrekte Verbindung der Lumina des ETT mit dem Ausatemschenkel und dem Einatemschenkel des Beatmungsgerätes sichergestellt ist. Grundsätzlich kann die korrekte Verbindung auch dadurch sichergestellt werden, daß plattenförmige Trennwände in Verbindungsstellung einander überlappen und azentrisch in Ansatzstück und Verbinder angeordnet sind. Ferner wird dies durch Trennwände in Form konzentrischer Kreisrohre sichergestellt, die eine beliebige Drehstellung zulassen.

Gemäß einer Ausgestaltung hat das Verschlußelement des Absperrorgans seinen Dichtsitz in der weiteren Trennwand und/oder der Trennwand. Durch Abrücken des

Verschlußelementes von seinem Dichtsitz wird die Trennwand geöffnet. Bei dem Absperrorgan handelt es sich z.B. um einen Ventilkegel oder eine Klappe.

Gemäß einer Ausgestaltung ist ein Bedienelement des Absperrorganes von außen manuell betätigbar. Die Betätigung von Hand kann durch medizinisches Personal erfolgen. Gemäß einer Ausgestaltung hat das Absperrorgan eine Wirkverbindung mit einer Steuerungseinrichtung des Beatmungsgerätes. Die Steuerung des Absperrorganes durch den Respirator ist sehr zuverlässig implementierbar. Gemäß einer Ausgestaltung weist das Absperrorgan eine Wirkverbindung mit einem Drucksensor im Einatmungs- oder Ausatmungszweig auf, z.B. in den Beatmungsschläuchen. Dort ist der Druck in einer normalen Bearbeitungssituation immer mehr oder weniger positiv. Bei negativem Druck ist hingegen eine Fehlersituation gegeben. Bei dieser Lösung kann eine unzuverlässige Betätigung von Hand und eine Modifikation des Respirators vermieden werden. Grundsätzlich sind die verschiedenen Steuerungen des Absperrorganes beliebig kombinierbar.

Gemäß einer Ausgestaltung weist das Absperrorgan eine Markierung auf, die jederzeit von außen sichtbar anzeigt, ob die beiden Lumina des Doppel-ETT bestimmungsgemäß phasengetrennt oder zugleich durchströmt werden. Gemäß einer Ausgestaltung weist das Absperrorgan einen Positionsgeber für die Position eines Verschlußelementes auf, der eine Wirkverbindung mit einer Steuerungseinrichtung des Beatmungsgerätes aufweist. Hierbei wird z.B. die Position des Absperrorgans automatisch bei der Steuerung der Beatmung und/oder einer Anzeige von Betriebsdaten und/oder von Alarmzuständen berücksichtigt.

Gemäß einer Ausgestaltung weist der Verbinder um mindestens eine Achse schwenkbare Anschlußgelenke für die Schläuche auf Dies ermöglicht ein Heranführen der Schläuche an den Verbinder in einem großen Winkelbereich. Es kann sich beispielsweise um Dreh- oder Schwenkgelenke mit einer oder mehreren Dreh- oder Schwenkachsen oder um Kugelgelenke handeln.

Gemäß einer Ausgestaltung weist der Verbinder eine Öffnung mit einem Verschluß auf, die geöffnet das Einführen eines Absaugkatheters in das exspiratorische Lumen des Endotrachealtubus ermöglicht. Durch die Verbindung ist bei laufender Beatmung ein Absaugkatheter in das exspiratorische Lumen des Doppel-ETT einführbar. Gemäß einer einfachen Ausgestaltung weist der Verschluß einen Stöpsel auf, der abdichtend in die Öffnung einsetzbar ist. Gemäß einer Ausgestaltung weist die Öffnung eine elastische Lochmembran auf, durch die das Absaugkatheter abdichtend einführbar ist, um den wünschenswerten positiven Druck in den Lungen zu halten. Die elastische Lochmembran ermöglicht eine Anpassung an Katheter verschiedenen Durchmessers.

Nachfolgend wird die Erfindung anhand der anliegenden Zeichnungen von Ausführungsbeispielen näher erläutert. In den Zeichnungen zeigen:
- Fig. 1 eine: Vorrichtung zur Beatmung in einer grobschematischen Ansicht;
- Fig. 2a und b: Verbindung zwischen Schlauchsystem und ETT derselben Vorrich- tung in einem vergrößerten, teilweisen Längsschnitt (Fig. 2a) und in einem Querschnitt durch den Verbindungsbereich (Fig. 2b);
- Fig. 3a und b: eine andere Verbindung zwischen ETT und Schlauchsystem in einem teilweisen Längsschnitt (Fig. 3a) und in einem Querschnitt durch den Verbindungsbereich (Fig. 3b);
- Fig. 4a und b: eine andere Verbindung zwischen ETT und Schlauchsystem in einem teilweisen Längsschnitt (Fig. 4a) und in einem Querschnitt durch den Verbindungsbereich (Fig. 4b);
- Fig. 5a und b: eine andere Verbindung zwischen ETT und Schlauchsystem in einem teilweisen Längsschnitt (Fig. 5a) und in einem Querschnitt durch den Verbindungsbereich (Fig. 5b).

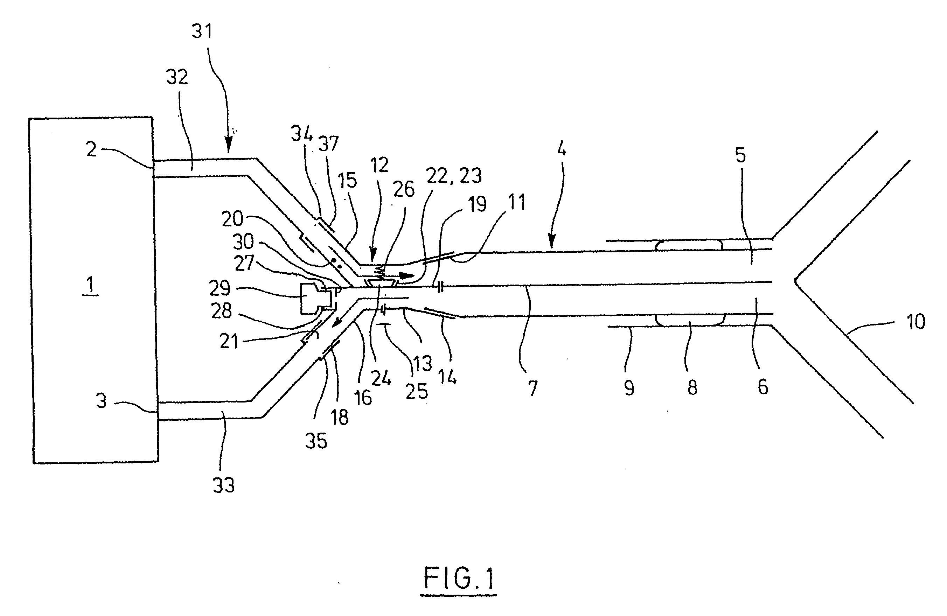

Gemäß Fig. 1 umfaßt die Vorrichtung zur Beatmung ein Beatmungsgerät 1 mit einem Ausgang 2, an dem ein Gasstrom zur Beatmung bereitgestellt wird. Ferner hat das Beatmungsgerät 1 einen Eingang 3, dem ein ausgeatmeter Gasstrom zugeführt werden kann.

Das Beatmungsgerät 1 hat im Eingang 3 ein aktives Ventil, das beim Einatmen geschlossen wird, um den Druck in der Lunge zu halten, und das beim Ausatmen geöffnet wird. Hinter dem Ventil kann das Ausatmungsgas der Umgebung zugeführt werden. Statt dessen kann im Beatmungsgerät 1 der ausgeatmete Gasstrom aufgearbeitet (CO₂) und das für die Beatmung benötigte Gas erneut dem Ausgang 2 zugeführt werden, wobei frisches Beatmungsgas zugemischt werden kann.

Ferner ist ein Endotrachealtubus 4 vorhanden, der in einem röhrenförmigen Körper zwei Lumina 5, 6 aufweist, die voneinander durch eine axiale Wand 7 getrennt sind. Am trachealen Ende hat dieser doppellumige ETT 4 eine aufblasbare Manschette 8, die zu einer Luftröhre 9 hin abdichtet, wenn der ETT 4 bis kurz vor die Bronchien 10 vorgeschoben ist.

Am patientenfernen Ende weist der ETT 4 ein Ansatzstück 11 in Form eines Konus auf. Die Trennwand 7 des ETT ist bis in das Ansatzstück 11 hinein verlängert, wobei sie etwa auf halber Höhe des Konus endet. Ferner ist ein Y-förmiger Verbinder 12 vorhanden. Dieser hat in einem im wesentlichen rohrförmigen Verbindungsabschnitt 13 eine konische Aufnahme 14, die abdichtend auf das Ansatzstück 11 aufgesetzt ist.

Der Verbindungsabschnitt 13 trägt zwei auseinanderstrebende Rohrstutzen 15, 16 mit Schlauchanschlüssen 17, 18 an den Enden. Ferner hat der Verbinder 12 eine weitere Trennwand 19, die sich etwa von der Mitte der konischen Aufnahme 14 aus axial bis zum Schnittpunkt der beiden Rohrstücke 15, 16 erstreckt. Hierdurch ist der Verbinder 12 in zwei Kammern geteilt, von denen die eingangsseitige 20 vom Schlauchanschluß 17 bis zur Aufnahme 14 und die ausgangsseitige 21 von der Aufnahme 14 bis zum Schlauchanschluß 18 erstreckt ist.

In der weiteren Trennwand ist ein Absperrorgan 22 mit einem Ventilsitz 23 und einem Kegelventil 24 angeordnet. Es ist mittels eines nach außen geführten Betätigungsknopfes 25 entgegen der Wirkung einer Rückstellfeder 26 öffenbar.

Die ausgangsseitige Kammer 21 weist zwischen den Rohrstücken 15, 16 ein Rohrstück 27 auf, das axial auf den rohrförmigen Verbindungsabschnitt 13 ausgerichtet ist und eine Öffnung 28 hat. In die Öffnung 28 ist ein Stöpsel 29 eingesetzt. Darunter befindet sich in dem Rohrstück 27 eine elastische Lochmembran 30.

Das Beatmungsgerät 1 ist über ein Schlauchsystem 31 über den Verbinder 12 mit dem ETT 4 verbunden. Das Schlauchsystem 31 hat einen Einatmungsschlauch 32, der mit dem Ausgang 2 und dem Schlauchanschluß 17 verbunden ist. Hierdurch ist der Ausgang 2 mit dem inspiratorischen Lumen 5 des ETT 4 verbunden. Ferner hat das Schlauchsystem 31 einen Ausatmungsschlauch 33, der mit dem Schlauchanschluß 18 verbunden ist. Im Beispiel sind die Verbindungen 34, 35 der Schläuche 32, 33 mit den Schlauchanschlüssen 17, 18 frei drehbar um die Achsen der Rohrstücke 15, 16 ausgeführt.

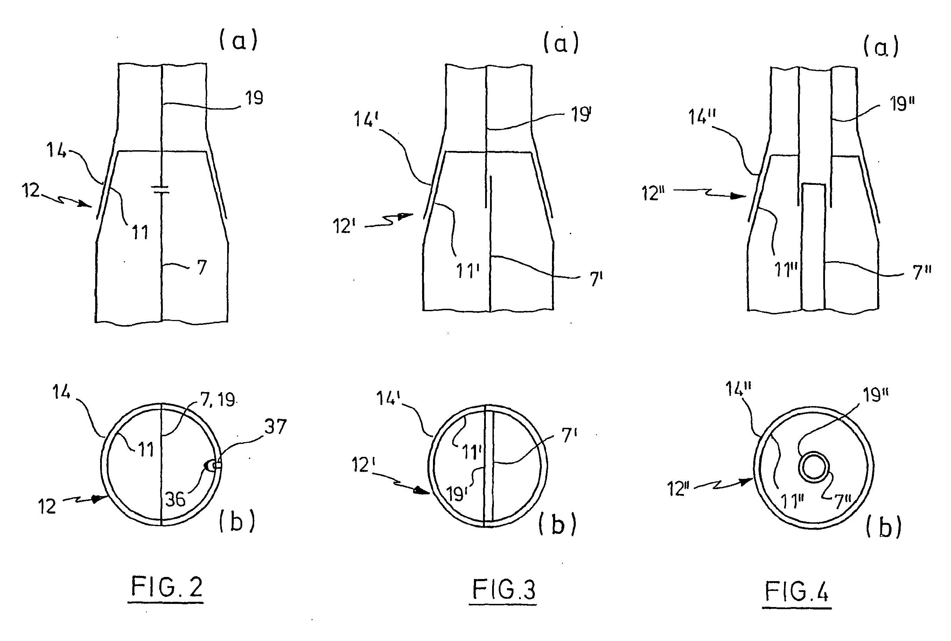

Die Fig. 2 zeigt, daß die Trennwand 7 und die weitere Trennwand 19 diagonal durch das Ansatzstück 11 und die Aufnahme 14 verlaufen. Wenn das Ansatzstück 11 dichtend in der Aufnahme 14 sitzt, stoßen sie stumpf und abdichtend gegeneinander. Die abdichtende Verbindung zwischen Ansatzstück 11 und Aufnahme 14 ist in nur einer Drehstellung, möglich, die durch eine axiale Nut 36 in der Außenseite des Ansatzstückes 11 und einen axialen Vorsprung 37 an der Innenseite der Aufnahme 14 vorgegeben ist. Hierdurch ist gewährleistet, daß die Kammer 20 stets mit dem Lumen 5 und die Kammer 21 stets mit dem Lumen 6 verbunden ist.

Somit ist die Verbindung des Beatmungsgerätes 1 mit dem ETT 4 durch einfaches Aufstecken des einzigen Verbinders 12 auf das Ansatzstück 11 möglich. Es ist sichergestellt, daß der Ausgang 2 mit dem inspirationsseitigen Lumen 5 und der Ausgang 3 mit dem exspirationsseitigen Lumen 6 verbunden ist, die unterschiedliche Querschnitte aufweisen. Im Bedarfsfall kann auch ein herkömmlicher Y-Verbinder mit dem ETT 4 verbunden werden.

Nach Entfernen des Stöpsels 29 ist ein Absaugkatheter abdichtend durch die Lochmembran 30 in das exspirationsseitige Lumen 6 bis vor die Bronchien 10 einschiebbar.

Die Ausführung von Fig. 3 unterscheidet sich von der vorbeschriebenen dadurch, daß die Trennwand 7' und die weitere Trennwand 19` etwas aus dem Zentrum herausgerückt sind und seitlich abdichtend aneinander liegen, wenn die Aufnahme 14 auf das Ansatzstück 11 gesetzt ist. Durch diese Anordnung wird zugleich gewährleistet, daß Verbinder 12` und Ansatzstück 11 nur in einer bestimmten Ausrichtung zusammengefügt werden können.

Die Ausführung von Fig. 4 unterscheidet sich von den vorbeschriebenen dadurch, daß die Trennwand 7" und die weitere Trennwand 19" jeweils als axial gerichtetes Rohr ausgeführt sind. Vorzugsweise ist das Rohr 7" mit dem inspirationsseitigen Lumen 5 und das Rohr 19" mit dem Schlauchanschluß 17 verbunden. Der umgebende Ringraum des Ansatzstückes 11 ist mit dem exspirationsseitigen Lumen 6 und der Aufnahme 14 mit dem Schlauchanschluß 18 verbunden. Die so ausgestalteten Verbinder 12" und Ansatzstücke 11 können in beliebiger Drehstellung zusammengesetzt werden.

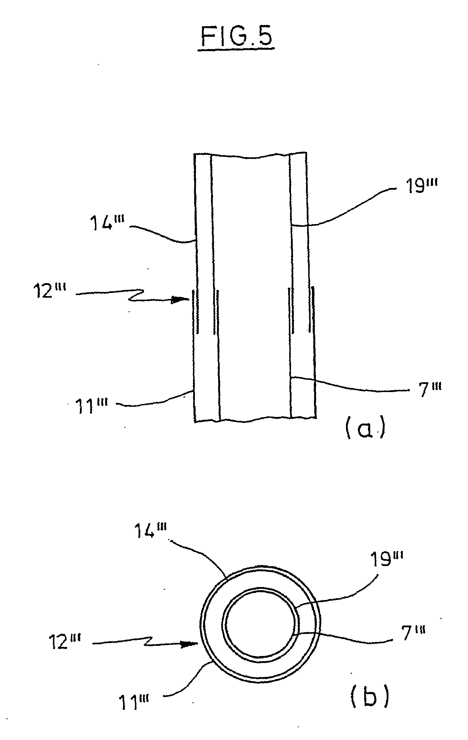

Die Ausführungen von Fig. 5 unterscheidet sich von der vorbeschriebenen dadurch, daß das Ansatzstück 11"' und die Aufnahme 14"' im Verbindungsbereich zylindrisch sind. Ferner dadurch, daß die Aufnahme 14" in das Ansatzstück 11'" eingesetzt ist. Dafür hat das Ansatzstück 14'" einen Außendurchmesser von etwa 22 mm und das Ansatzstück 11'" einen entsprechenden Innendurchmesser.

Das Rohr 7"` ist wie bei der vorbeschriebenen Ausführung in das Rohr 19"' eingesteckt, wobei die beiden Rohre 7"', 19'" einen größeren Außen- bzw. Innendurchmesser von etwa 15 mm aufweisen.

Vorzugsweise wird das Innenlumen der Rohre 7"', 19" für die Exspiration und das ringförmige Außenlumen zwischen den Rohren 7'", 19'" und Ansatzstück 11'" sowie Aufnahme 14'" für die Inspiration genutzt.

## Patentansprüche

1. Vorrichtung zur Beatmung mit
- einem doppellumingen Endotrachealtubus (4) mit einem Ansatzstück (11) am patientenfernen Ende, in das die beiden Lumina (5, 6) des Endotrachealtubus (4) hineingeführt sind, wobei sie im Ansatzstück (11) durch eine axiale Trennwand (7) voneinander getrennt sind,
- einem Verbinder (12) mit zwei Schlauchanschlüssen (17, 18), die in einer Aufnahme (14) für das Anschlußstück (11) zusammengeführt und durch eine weitere axiale Trennwand (19) voneinander getrennt sind, der mit dem Ansatzstück (11) unter abdichtender Anlage der Trennwand (7) und der weiteren Trennwand (19) aneinander verbindbar ist,
- einem Beatmungsgerät (1) und
- mindestens einem einenends mit einem Schlauchanschluß (17, 18) des Verbinders (12) und anderenends mit dem Beatmungsgerät (1) verbundenen Schlauch (32, 33), **dadurch gekennzeichnet dass**
• der Verbinder (12) und/oder das Ansatzstück (11) ein Absperrorgan (22) aufweist/aufweisen, das die beiden Seiten der weiteren Trennwand (19) und/oder der Trennwand (7) überbrückt.

2. Vorrichtung nach Anspruch 1, bei der das Ansatzstück (11) einen kreisförmigen Außenumfang und die Aufnahme (14) einen entsprechenden Innenumfang aufweist.

3. Vorrichtung nach Anspruch 1 oder 2, bei der das Ansatzstück (11) und die Aufnahme (14) konisch oder zylindrisch sind.

4. Vorrichtung nach einem der Ansprüche 1 bis 3, bei der der Verbinder (12) Y- oder T-förmig ist.

5. Vorrichtung nach einem der Ansprüche 1 bis 4, bei der die Trennwände (7, 19) bei abdichtender Anlage stumpf gegeneinanderstoßen oder einander teilweise überlappen.

6. Vorrichtung nach einem der Ansprüche 1 bis 5, bei der die Trennwände (7, 19) plattenförmig oder rohrförmig sind.

7. Vorrichtung nach einem der Ansprüche 1 bis 6, bei der das Ansatzstück (11"") oder eine darin angeordnete rohrförmige Trennwand (7"') einen Außendurchmesser von etwa 15 mm und die Aufnahme (144") oder eine darin angeordnete weitere rohrförmige Trennwand (19"') einen Innendurchmesser von etwa 15 mm aufweisen und/oder bei der das Ansatzstück (11"') einen Innendurchmesser von etwa 22 mm und die Aufnahme (14"') einen Außendurchmesser von etwa 22 mm aufweisen.

8. Vorrichtung nach einem der Ansprüche 1 bis 7, bei der das Ansatzstück (11) mindestens eine Nut (36) und der Verbinder (12) mindestens einen komplementären Vorsprung (37) aufweist oder umgekehrt, die bei Verbindung von Ansatzstück (11) und Verbinder (12) ineinandergreifen.

9. Vorrichtung nach Anspruch 1, **dadurch** gekennzeichet, daß ein Verschlußelement (24) des Absperrorganes (22) einen Dichtsitz (23) in der weiteren Trennwand (19) und/oder der Trennwand (7) hat.

10. Vorrichtung nach Anspruch 1, **dadurch gekennzeichnet, daß** bei der das Verschlußelement (24) ein von außerhalb des Verbinders (12) und/oder des Ansatzstückes (11) manuell betätigbares Betätigungselement (25) aufweist.

11. Vorrichtung nach einem der Ansprüche 1 bis 10, bei der das Absperrorgan (22) eine Wirkverbindung mit einer Steuerungseinrichtung des Beatmungsgerätes (1) aufweist.

12. Vorrichtung nach einem der Ansprüche 1 bis 11, bei der das Absperrorgan (22) eine Wirkverbindung unit einem Drucksensor im Einatmungs- oder Ausatmungszweig aufweist.

13. Vorrichtung nach einem der Ansprüche 1 bis 12, bei der das Absperrorgan (22) einen Positionsgeber für die Position eines Verschlußelementes (24) hat, der eine Wirkverbindung mit einer Steuerungseinrichtung des Beatmungsgerätes (1) aufweist.

14. Vorrichtung nach einem der Ansprüche 1 bis 13, bei der der Verbinder (12) um mindestens eine Achse schwenkbare Anschlußgelenke (34, 35) für die Schläuche (32, 33) aufweist.

15. Vorrichtung nach einem der Ansprüche 1 bis 14, bei der der Verbinder (12) eine Öffnung (28) mit einem Verschluß (29) aufweist, die geöffnet das Einführen eines Absaugkatheters in das exspiratorische Lumen (6) des Endotrachealtubus (4) ermöglicht.

16. Vorrichtung nach Anspruch 15, bei der der Verschluß (29) einen Stöpsel aufweist, der abdichtend in die Öffnung (28) einsetzbar ist.

17. Vorrichtung nach Anspruch 15 oder 16, bei der die Öffnung (28) eine elastische Lochmembran (30) umfaßt, durch die das Absaugkatheter abdichtend einführbar ist.

## Claims

1. Device for ventilation with
- a double lumen endotracheal tube (4) with a connecting piece (11) at the end distal to the patient, both lumina (5, 6) of the endotracheal tube (4) are introduced into the connecting piece (11) and separated within it by an axial partition wall (7),
- a connector (12) with two tubing connections (17, 18) being joined in an opening (14) accepting the connecting piece (11) and separated from each other by another partition wall (19), connectable to the connection piece (11) with the partition wall (7) and the other partition wall (19) joining to form a seal,
- a ventilator (1), and
- at least one tube (32, 33) connected to the tubing connections (17, 18) of the connector (12) at one end and to the ventilator (1) at the other, **characterized in that**
- the connector (12) and / or the connecting piece (11) feature a shutter (22) bridging the two sides of the other partition wall (19) and / or the partition wall (7).

2. Device according to claim 1, in which the connecting piece (11) features a circular external circumference and the opening (14) features a corresponding internal circumference.

3. Device according to claims 1 or 2, in which the connecting piece and the opening (14) are conical or cylindrical.

4. Device according to one of the claims 1 through 3, in which the connector (12) is Y- or T-shaped.

5. Device according to one of the claims 1 through 4, in which the partition walls (7, 19) meet bluntly or partially overlap for forming a seal.

6. Device according to one of the claims 1 through 5, in which in which the partition walls (7, 19) are shaped like plates or tubes.

7. Device according to one of the claims 1 through 6, in which the connecting piece (11"`) or a tube-shaped partition wall (7"`) located within it has an external diameter of approximately 15mm and the opening (14"') or the other tub-shaped partition wall (19'") located within it has an internal diameter of 15mm and / or in which the connecting piece (11"') has an internal diameter of approximately 22mm and the opening (14"') has an external diameter of approximately 22mm.

8. Device according to one of the claims 1 through 7, in which the connecting piece (11) features at least one groove and the connector (12) has at least one corresponding projection or vice versa, which articulate when connecting piece (11) and connector (12) are joined.

9. Device according to claim 1, in which a closing element (24) in or on the shutter (22) has its seat (23) in the other partition wall (19) and / or the partition wall (7).

10. Device according to claim 1, in which the closing element (24) has an operating element (25) to be operated manually from outside the connector (12) and / or the connecting piece (11).

11. Device according to one of the claims 1 through 10, in which the shutter (22) features an operating connection with a control component of the ventilator (1).

12. Device according to one of the claims 1 through 11, in which the shutter (22) features an operating connection with a pressure sensor in the inspiratory or the expiratory limb.

13. Device according to one of the claims 1 through 12, in which the shutter (22) has a sensor for the position of the closing element (24) featuring an operating connection to a control component of the ventilator (1).

14. Device according to one of the claims 1 through 13, in which the connector (12) features joints (34, 35) for the tubes (32,33) which can be swiveled around at least one axis.

15. Device according to one of the claims 1 through 14, in which the connector (12) has an opening (28) with a fastener (29) which, when opened, allows the introduction of a suction catheter into the expiratory lumen (6) of the endotracheal tube (4).

16. Device according to claim 15, in which the fastener (29) features a plug to be introduced into and forming a seal with the opening (28).

17. Device according to claims 15 or 16, in which the opening (28) includes an elastic perforated membrane (30), through which the suction catheter can be introduced while maintaining an airtight seal around it.

## Revendications

1. Dispositif de respiration artificielle comportant
- un tube endotrachéal (4) à deux canaux, avec un embout (11) à l'extrémité éloignée du patient et dans lequel les deux canaux (5, 6) du tube endotrachéal (4) sont introduits, ces canaux étant séparés l'un de l'autre dans l'embout (11) par une paroi de séparation (7) axiale,
- un raccord (12), avec deux raccordements de tuyau (17, 18) qui sont introduits ensemble dans un logement (14) de l'embout (11) et qui sont séparés l'un de l'autre par une autre paroi de séparation (19) axiale, et qui est raccordé à l'embout (11) en rapprochement étanche l'une de l'autre de la paroi de séparation (7) et de l'autre paroi de séparation (19),
- un appareil de respiration artificielle (1) et
- au moins un tuyau (32, 33) raccordé, à une extrémité, à un raccordement de tuyau (17, 18) du raccord (12) et, à l'autre extrémité, à l'appareil de respiration artificielle (1),
**caractérisé en ce que**
• le raccord (12) et/ou l'embout (11) présente/présentent un organe d'arrêt (22) qui enjambe les deux côtés de l'autre paroi de séparation (19) et/ou de la paroi de séparation (7).

2. Dispositif selon la revendication 1, dans lequel l'embout (11) présente une circonférence extérieure annulaire, et le logement (14) une circonférence intérieure correspondante.

3. Dispositif selon la revendication 1 ou 2, dans lequel l'embout (11) et le logement (14) sont coniques ou cylindriques.

4. Dispositif selon une des revendications 1 à 3, dans lequel le raccord (12) est en forme de Y ou de T.

5. Dispositif selon une des revendications 1 à 4, dans lequel les parois de séparation (7, 19), en rapprochement étanche, viennent s'abouter ou se chevauchent partiellement.

6. Dispositif selon une des revendications 1 à 5, dans lequel les parois de séparation (7, 19) sont en forme de plaque ou de tube.

7. Dispositif selon une des revendications 1 à 6, dans lequel l'embout (11"') ou une paroi de séparation (7"') tubulaire qui y est disposée présente un diamètre extérieur d'environ 15 mm, et le logement (14"') ou une autre paroi de séparation (19"') tubulaire qui y est disposée présente un diamètre intérieur d'environ 15 mm, et/ou dans lequel l'embout (11"') présente un diamètre intérieur d'environ 22 mm, et le logement (14"') un diamètre extérieur d'environ 22 mm.

8. Dispositif selon une des revendications 1 à 7, dans lequel l'embout (11) présente au moins une rainure (36), et le raccord (12) au moins une saillie (37) complémentaire ou inversement, qui entrent en prise l'une avec l'autre lors du raccordement de l'embout (11) et du raccord (12).

9. Dispositif selon la revendication 1, **caractérisé en ce qu'**un élément de fermeture (24) de l'organe d'arrêt (22) a un siège d'étanchéité (23) dans l'autre paroi de séparation (19) et/ou dans la paroi de séparation (7).

10. Dispositif selon la revendication 1, **caractérisé en ce que** l'élément de fermeture (24) présente un élément d'actionnement (25) pouvant être actionné manuellement depuis l'extérieur du raccord (12) et/ou de l'embout (11).

11. Dispositif selon une des revendications 1 à 10, dans lequel l'organe d'arrêt (22) présente un lien opératoire avec un dispositif de commande de l'appareil de respiration artificielle (1).

12. Dispositif selon une des revendications 1 à 11, dans lequel l'organe d'arrêt (22) présente un lien opératoire avec un manomètre dans la branche d'inspiration ou d'expiration.

13. Dispositif selon une des revendications 1 à 12, dans lequel l'organe d'arrêt (22) a un transmetteur de position pour la position d'un élément de fermeture (24) qui présente un lien opératoire avec un dispositif de commande de l'appareil de respiration artificielle (1).

14. Dispositif selon une des revendications 1 à 13, dans lequel le raccord (12) présente, pour les tuyaux (32, 33), des articulations de raccordement (34, 35) pouvant pivoter autour d'au moins un axe.

15. Dispositif selon une des revendications 1 à 14, dans lequel le raccord (12) présente une ouverture (28) avec une obturation (29) qui, quand elle est ouverte, permet l'introduction d'un cathéter d'aspiration dans le canal expiratoire (6) du tube endotrachéal (4).

16. Dispositif selon la revendication 15, dans lequel l'obturation (29) présente un bouchon qui peut être inséré de façon étanche dans l'ouverture (28).

17. Dispositif selon la revendication 15 ou 16, dans lequel l'ouverture (28) comprend une membrane perforée (30) élastique à travers laquelle le cathéter d'aspiration peut être introduit de façon étanche.
